(19) **Europäisches Patentamt**
**European Patent Office**
**Office européen des brevets**

(11) **EP 2 753 298 B1**

(12) **EUROPEAN PATENT SPECIFICATION**

(45) Date of publication and mention
of the grant of the patent:
**16.03.2016 Bulletin 2016/11**

(21) Application number: **12758442.3**

(22) Date of filing: **04.09.2012**

(51) Int Cl.:
*A61K 8/34* *(2006.01)*     *A61K 8/35* *(2006.01)*
*A61K 8/37* *(2006.01)*     *A61K 8/81* *(2006.01)*
*A61K 8/87* *(2006.01)*     *A61Q 5/06* *(2006.01)*

(86) International application number:
**PCT/EP2012/067152**

(87) International publication number:
**WO 2013/034527 (14.03.2013 Gazette 2013/11)**

(54) **HAIR FIXATIVE COMPOSITIONS**

HAARFESTIGERZUSAMMENSETZUNGEN

COMPOSITIONS DE FIXATION POUR CHEVEUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.09.2011 US 201161531832 P
20.01.2012 EP 12151942**

(43) Date of publication of application:
**16.07.2014 Bulletin 2014/29**

(73) Proprietor: **Akzo Nobel Chemicals International
B.V.
6824 BM Arnhem (NL)**

(72) Inventors:
• **BELLUSCIO, Maryalice
Long Valley, New Jersey 07853 (US)**
• **HE, Qiwei
Belle Mead, New Jersey 08502 (US)**

(74) Representative: **Akzo Nobel IP Department
Velperweg 76
6824 BM Arnhem (NL)**

(56) References cited:
**EP-A1- 2 213 333     WO-A1-2010/116951**

**Description**

FIELD OF THE INVENTION

The present invention relates to hair fixative compositions. More specifically, the present invention relates to hair fixative compositions including fixative polymers and solvent systems comprising the combination of ethanol and at least one other non-aqueous solvent.

BACKGROUND OF THE INVENTION

Governmental agencies, such as the California Air Resource Board, are currently considering using the Maximum Incremental Reactivity (MIR) scale to rank the ozone-forming potential of all volatile organic compounds (VOCs). Consequently, the current consumer product regulation for hair fixatives containing VOCs, such as hairsprays, would be modified from the current VOC scale to the MIR scale. Although no MIR Value for hairsprays has yet been adopted, some proposals would require hairspray compositions to have a MIR value of less than 0.80. Accordingly, if an MIR of 0.80 or less is implemented, reformulation of current anhydrous 55% VOC aeorosol hairsprays and all non-aerosol hairsprays would be required to comply with the new regulations. Accordingly, there is a need for hairspray formulations that not only meet the more stringent MIR requirements, but that meet or exceed the performance of conventional hairspray formulations.

WO2010/11695 relates to a hair cosmetic comprising an adhesive setting resin and further comprising at least one member selected from a cationic surfactant, a silicone derivative, and a polyhydric alcohol, and an alcohol.

EP2213333 relates to a hair cosmetic composition with water content of up to 10 wt.%, packaged in an aerosol device, and that comprises one or more anionic fixing polymers; one or more polyols; one or more liquid fatty alcohols; one or more lower 1-4C monoalcohol; and one or more propellants.

SUMMARY OF THE INVENTION

In an aspect of the present invention, the present invention generally relates to a hair fixative composition comprising at least one fixative polymer and ethanol in combination with at least one non-aqueous solvent selected from the group consisting of iso-propanol, n-propanol and combinations thereof, in a solvent system wherein the at least one fixative polymer is selected from the group consisting of octylacrylamide/acrylates/butylaminoethyl methacrylate copolymer), acrylates/octylacrylamide copolymer, acrylates copolymer, octylacrylamide/butylaminoethyl methacrylate copolymer, VA/crotonates/vinyl neodecanoate copolymer and polyurethane-14 (and) AMP-Acrylates copolymer. In an embodiment, the solvent system comprises ethanol and the at least one other non-aqueous solvent in a weight ratio in a range of about 80:20 to about 20:80 of ethanol to the at least one other non-aqueous solvent.

In a specific embodiment, the present invention is directed to the hair fixative composition comprising the at least one fixative polymer, solvent system, and further a propellant.

BRIEF DESCRIPTION OF THE DRAWING

The invention is best understood from the following detailed description when read in connection with the accompanying drawings. Included in the drawings are the following figures:

Figure 1 is a chart depicting the viscosity of example hair fixative compositions including several different polymers and solvent systems of varying concentrations.
Figure 2 is a chart depicting the turbidity of example hair fixative compositions including several different polymers and solvent systems of varying concentrations.
Figure 3 is a chart depicting the surface tension of example hair fixative compositions including several different polymers and solvent systems of varying concentrations.
Figure 4 is a chart depicting the capillary number of example hair fixative compositions including several different polymers and solvent systems of varying concentrations.
Figure 5 is a graph depicting the particle size of example hair fixative compositions including several different polymers and solvent systems of varying concentrations.
Figure 6 is a chart depicting high humidity curl retention of example hair fixative compositions including two different polymers and solvent systems of varying concentrations.
Figure 7 is a graph illustrating polymer film elongation of hair fixative polymer films derived from hair fixative compositions including two different AMPHOMER® polymers and solvent systems of varying concentrations.

DETAILED DESCRIPTION OF THE INVENTION

The present invention generally relates to a hair fixative composition comprising at least one fixative polymer and a solvent system comprising one or more non-aqueous solvents, selected from the group consisting of iso-propanol, n-propanol and combinations thereof, in combination with ethanol wherein the at least one fixative polymer is selected from the group consisting of octylacrylamide/acrylates/butylaminoethyl methacrylate copolymer), acrylates/octylacrylamide copolymer, acrylates copolymer, octylacrylamide/butylaminoethyl methacrylate copolymer, VA/crotonates/vinyl neodecanoate copolymer and polyurethane-14 (and) AMP-Acrylates copolymer. In an embodiment, the solvent system will comprise ethanol in combination with the at least one other non-aqueous solvent, such that the hair fixative composition has a MIR value of about 0.80 or less. Optionally, water may also be included in the hair fixative composition.

It has been found that a solvent system comprising ethanol and the one or more additional non-aqueous solvents in a range of ratios of 80:20 to 20:80 of ethanol to the other one or more non-aqueous solvents, provides a heretofore unknown improvement to long term hold and durability of the resultant dry hair fixative polymer film, particularly where it was unexpected that the selection of solvents would have any significant impact on the on-hair performance properties of the hair fixative polymer film after the film was allowed to dry.

For purposes of this invention, propellants are not included as part of the solvent system as defined herein, although propellants may optionally be included as components of the hair fixative composition. In an aspect of the invention, it has been found that solvent systems comprising ethanol and at least one other non-aqueous solvent in weight ratios ranging from about 80:20 to about 20:80 of ethanol to the other solvent have improved on hair performance properties, such as long term hold and durability of the resultant dry hair fixative polymer film. In particular, the present invention provides an improvement in subjective hair properties, such as stiffness, spring, and webbing, to hair treated with the hair fixative composition, while also maintaining a level of % curl retention of the hair under high humidity conditions (21.1°C/90% Relative Humidity) compared to conventional hair fixative compositions comprising only one non-aqueous solvent alone.

In an embodiment of the invention, the solvent system of the hair fixative composition has a weight ratio in a range of about 80:20 to about 20:80 of ethanol to at least one other non-aqueous solvent. In a further embodiment, the hair fixative composition has a weight ratio in a range of about 75:25 to about 25:75. In yet another embodiment, the weight ratio is from about 70:30 to about 30:70, in a further embodiment a weight ratio of about 65:35 to about 35:65, and in still yet another embodiment a weight ratio of about 60:40 to about 40:60. In another embodiment, the ratios are from greater than 50:50 to about 75:25.

In an embodiment, the solvent system used in the hair fixative composition comprises about 40% to about 98% by weight of the hair fixative composition, and in another embodiment, about 60% to about 95% by weight.

In addition to the solvent system, the hair fixative composition includes the at least one fixative polymer. The polymers of this invention can be anionic or amphoteric copolymers. Non-limiting examples of these additional hair fixative polymers include: from Akzo Nobel Surface Chemistry LLC, AMPHOMER® 4961 and AMPHOMER® LV-71 polymers (octylacrylamide/acrylates/butylaminoethyl methacrylate compolymer), AMPHOMER® HC polymer (acrylates/octylacrylamide copolymer) BALANCE® 0/55 and BALANCE® CR polymers (acrylates copolymer), BALANCE® 47 polymer (octylacrylamide/butylaminoethyl methacrylate copolymer), RESYN® 28-2930 polymer (VA/crotonates/vinyl neodecanoate copolymer), DynamX polymer (polyurethane-14 (and) AMP-Acrylates copolymer), RESYN® XP polymer (acrylates/octylacrylamide copolymer); water-soluble acrylics; water-soluble polyesters;; and other conventional polymer that is polar solvent soluble or that can be made soluble through neutralization with the appropriate base. The official chemical description of each of these chemical names can be found in the INCI dictionary or at the website (www.ctfa.org). In an embodiment of the invention, the hair fixative polymer is selected from octylacrylamide/Acrylates/Butylaminoethyl Methacrylate Copolymer, polyurethane-14(and)acrylates copolymer and VA/crotonates/vinyl neodecanoate. A combination of one or more of the above hair fixative polymers is also contemplated as within the scope of the present invention.

In a further embodiment, the hair fixative polymers of the invention comprise copolymers formed from one or more monomers. Non-limiting examples of monomers suitable for use in the present invention are acrylic acid, methacrylic acid, methylacrylate, methacrylate, vinyl acetate, crotonic acid, vinyl neodecanate, isocyanate, t-octylacrylamide and the like. In the case where the monomer is acrylic or methacrylic acid, the acid group can be neutralized with typical reagents such as triethanol amine (TEA), AMP, sodium carbonate, sodium hydroxide or the like. In one embodiment of this invention the polymer is a copolymer of acrylate or methacrylate monomers.

In an embodiment of the invention, the fixative polymer is present in the hair fixative compositions in an amount of about 0.1 to 10% by weight of the composition. In a further embodiment, the fixative polymer is present in an amount of about 1-10% by weight and in a further embodiment in an amount of about 1 to 7% by weight.

Another feature of the present invention is that the hair fixative composition has a Maximum Incremental Reactivity (MIR) of less than about 0.8 in an embodiment, and in further embodiments the MIR value is about 0.75 or less or about 0.70 or less.

MIR is defined as an incremental reactivity (IR) calculated for a volatile organic mixture where the emissions

of NO$_x$ (NO + NO$_2$) have been adjusted to maximize the calculated MIR. IR can be determined by the following formula (I):

$$IR = \Delta[O_3]/\Delta[VOC] \qquad\qquad (I)$$

Thus, for a specified set of meteorological conditions, emissions, and initial concentrations, the incremental reactivity of an organic compound is the change in the peak ozone concentration, $\Delta[O_3]$, in grams, divided by an incremental change in the initial concentration and emissions of the organic compound $\Delta[VOC]$, in grams. For a given volatile organic ingredient, its MIR has been determined and the MIR values can be obtained, for example from The California Environmental Protection Agency Air Resources Board at www.arb.ca.gov/research/reactivity/reactivity.htm.

Thus, to determine overall MIR value of the hair fixative compositions of the invention, the following formula was used:

$$Wtd\ MIR\ Ingredient = MIR\ x\ Weight\ Fraction\ Ing.$$

$$PWMIR = (Wtd\ MIR)1 + (Wtd\ MIR)2 + \ldots\ldots + (Wtd\ MIR)n \qquad (III)$$

where Wtd MIR ingredient is the weighted MIR value for each ingredient and PWMIR is the sum of the weighted MIR values of the ingredients of the composition.

In another embodiment, the hair fixative composition has a viscosity of about 0.10 Pascal seconds or less, in another embodiment a viscosity of about 0.080 Pascal seconds or less and in a further embodiment 0.060 or less Pascal seconds, and in an even further embodiment 0.050 or less Pascal seconds. For hairsprays in particular, it is important that the viscosity is not above 0.10 Pascal seconds, as it has been found that the more viscous (or thicker) the composition, the ability to spray the composition is hindered.

In an embodiment of the invention, the hair fixative composition in the solvent system has a turbidity of about 30 NTU or less, in another embodiment a turbidity of about 20 NTU or less, 15 NTU or less and in a further embodiment 10 NTU or less. For hairsprays, it is important that the turbidity is not above 30 NTU, as it has been found that the higher the turbidity the less soluble the polymer is in the solvent system, which in turn hinders the sprayability of the hairspray formulation.

In a further embodiment, the hair fixative composition in the solvent system has a surface tension of about 28 Dynes/cm or less in a further embodiment of about 21 Dynes /cm or less and in still yet a further embodiment of about 28 Dynes/cm to 10 Dynes/cm and another embodiment about 21 Dynes/cm to about 18 Dynes/cm. For hairsprays in particular, it is important that the surface tension is not above 28 Dynes/cm, as it has been found that the higher the surface tension the harder the solution is to spray.

In another embodiment, the hair fixative composition has a mean particle size after being atomized out of solvent system from about 40 to 125 microns, in another embodiment of about 60 to 90 microns and still further in an amount of about 55-80 microns.

In yet another embodiment, the hair fixative composition has a capillary number of about 0.005 or less, in yet another embodiment of about 0.002 or less pascal seconds/dynes/cm or less, and in another embodiment of about 0.00105 or less.

In an embodiment of the invention, the hair fixative compositions optionally further includes a neutralizing agent. In an embodiment of the invention, the fixative polymer is generally at least about 80% neutralized. In another embodiment, the fixative polymer is at least about 90% neutralized, and in an even further embodiment, the fixative polymer is 100% neutralized. Suitable basic neutralizing agents compatible with the composition can be employed, even inorganic materials such as sodium or potassium hydroxide. Generally organic amines or alkanolamines are readily used for neutralization. In an embodiment, the neutralizing agents include, but are not limited to aminomethylpropanol, and di- methyl stearamine, sodium hydroxide, potassium hydroxide and triethanolamine.. Inorganic materials, such as sodium or potassium hydroxide, may also be used. In an embodiment of the invention, the neutralizing agent is an organic amine or alkanolamine.

Other optional additives to provide certain modifying properties to the composition include, but are not limited to, silicones and silicone derivatives; humectants; moisturizers; plasticizers, such as glycerine, glycol and phthalate esters and ethers; emollients, lubricants and penetrants, such as lanolin compounds; fragrances and perfumes; UV absorbers; dyes, pigments and other colorants; anticorrosion agents; antioxidants; detackifying agents; combing aids and conditioning agents; antistatic agents; neutralizers; glossifiers; proteins, protein derivatives and amino acids; vitamins; emulsifiers; surfactants; viscosity modifiers; stabilizers; sequestering agents; chelating agents; aesthetic enhancers; fatty acids, fatty alcohols and triglycerides; botanical extracts; film formers; and clarifying agents. Such additives are commonly used in hair cosmetic compositions known heretofore. These additives are present in small, effective amounts to accomplish their function, and generally will comprise from about 0.01 to 10% by weight each, and from about 0.01 to 20% by weight total, based on the weight of the composition.

The compositions of the present invention may also optionally include one or more propellants. Thus, in another aspect, the invention provides a hair fixative composition comprising a fixative polymer in a solvent system in combination with a propellant. Such propellants include, without limitation, ethers, such as dimethyl ether; one or more lower boiling hydrocarbons such as $C_3$-$C_6$ straight and branched chain hydrocarbons, for example, propane, butane, and isobutane; halogenated hydrocarbons, such as, hydrofluorocarbons, for example, 1,1-difluoroethane and 1,1,1,2-tetrafluoroethane, present as a liquefied gas; and the compressed gases, for example, nitrogen, air and carbon dioxide. In an embodiment of the invention, the propellant is present in an amount of about 25% to about 60% by weight of the hair fixative composition including the solvent system. In a further embodiment, the propellant is present in an amount of about 30% to about 50 % by weight.

The hair fixative compositions of the present invention include, but are not limited, to aerosol and non-aerosol hairsprays.

In a further aspect of the invention, the invention provides a method for preparing a hair fixative composition. The method comprises dissolving a fixative polymer in a solvent system wherein the solvent system comprises ethanol and at least one other non-aqueous solvent selected from the group consisting of iso-propanol, n-propanol and combinations thereof in a weight ratio of about 80:20 to about 20:80 ethanol to the at least one other non-aqueous solvent. In an embodiment, the method further includes neutralizing the solution with a neutralizing agent, such as aminomethylpropanol. In a further embodiment, the fixative polymer is dissolved in a first non-aqueous solvent, next the solution is neutralized and then a second non-aqueous solvent, and optionally water, is added to the solution, wherein the first and second non-aqueous solvents are present in solvent system in a weight ratio of about 80:20 to about 20:80 of first non-aqueous solvent to second non-aqueous solvent.

Examples

The following examples are intended to exemplify the present invention but are not intended to limit the scope of the invention in any way. The breadth and scope of the invention are to be limited solely by the claims appended hereto.

Example 1 - Determination of MIR In Aerosol Spray Hair Fixatives

Samples of formulations containing a polymer and various solvent systems were tested in order to determine the MIR of the Samples tested. The formulations were prepared according to the following procedure:

Sample Preparation Procedure:

1. To the main mixing vessel charge all the ethanol contained in the formulation.
2. Begin mixing with propeller agitation (Adjust the speed of the speed of the mixing until there is a vortex pulled 2/3 of the way down the mixing shaft)
3. Slowly add the polymer powder by sifting it into the side of the vortex.
4. Allow the polymer to disperse completely, than add the Aminomethylpropanol(neutralizing agent).
5. Maintain mixing until the polymer is completely dissolved and the solution is clear.
6. Add the remaining solvents (Isopropanol and/or water)

Example aerosol and non-aerosol formulations are shown in Tables 1 and 2 below.

Table 1

| Aerosol Formulations | | | | | | | |
|---|---|---|---|---|---|---|---|
| Sample | A | B | C | D | E | F | G |
| Ingredient | Control (ETOH) | ETOH:water | IPA | IPA:ETOH 75:25 | IPA:water | ETOH:IPA 75:25 | IPA:ETOH 50:50 |
| Polymer | 5.00 | 5.00 | 5.00 | 5.00 | 5.00 | 5.00 | 5.00 |
| AMP | 0.96 | 0.96 | 0.96 | 0.96 | 0.96 | 0.96 | 0.96 |

(continued)

| Aerosol Formulations | | | | | | | |
|---|---|---|---|---|---|---|---|
| Sample | A | B | C | D | E | F | G |
| Water | 0.00 | 7.50 | 0.00 | 0.00 | 5.00 | 0.00 | 0.00 |
| Ethanol (ETOH) | 53.50 | 46.00 | 0.00 | 15.00 | 0.00 | 42.00 | 26.75 |
| Isopropanol (IPA) | 0.00 | 0.00 | 53.50 | 38.50 | 48.50 | 12.04 | 26.75 |
| Propellant (Hydroflurocarbon 152a) | 40.00 | 40.00 | 40.00 | 40.00 | 40.00 | 40.00 | 40.00 |
| MIR value | 0.78 | 0.68 | 0.32 | 0.45 | 0.29 | 0.69 | 0.55 |

Table 2

| Non-aerosol formulations | | | | | | | |
|---|---|---|---|---|---|---|---|
| Sample | A | B | C | D | E | F | G |
| Ingredient | Control (ETOH) | ETOH:water | IPA | IPA:ETOH 75:25 | IPA:water | ETOH:IPA 75:25 | IPA:ETOH 50:50 |
| Polymer | 5.00 | 5.00 | 5.00 | 5.00 | 5.00 | 5.00 | 5.00 |
| AMP | 0.96 | 0.96 | 0.96 | 0.96 | 0.96 | 0.96 | 0.96 |
| Water | 40 | 47.50 | 40.00 | 40.00 | 45.00 | 40.00 | 40.00 |
| Ethanol (ETOH) | 53.50 | 46.00 | 0.00 | 15.00 | 0.00 | 42.00 | 26.75 |
| Isopropanol (IPA) | 0.00 | 0.00 | 53.50 | 38.50 | 48.50 | 12.04 | 26.75 |
| | | | | | | | |
| MIR value | 0.78 | 0.68 | 0.32 | 0.45 | 0.29 | 0.69 | 0.55 |

Samples A-G were tested using various polymers, including AMPHOMER®, BALANCE® CR, RESYN® 28-2930, DynamX®, all available from Akzo Nobel Surface Chemistry LLC, Bridgewater, NJ, and Luvimere® 100P, LUVISET® CAN and LUVISET® PUR available from BASF and ACUDYNE™ 180 available from DOW Chemical. It was determined that the polymer had no effect on the overall MIR value. As shown in Tables 1 and 2, Samples B through G (with A being the control) using various solvent combinations all had an MIR value below about 0.70.

Example 2 - Determination of Viscosity

Samples of formulations containing several different hair fixative polymers in various solvent systems were tested in order to determine the viscosity of the sample formulations. The hairspray formulations were made by weighing up the solvents, agitating with an overhead mixer with propeller agitation. The polymers were slowly sifted into the solvent and allowed to mix until the polymers were completely dissolved in the solvent. Viscosity measurements were performed on a Dynamic Stress Rheometric manufactured by Rheometric Scientific Rheometer model SR 5000. The Couette fixture, having a Cup diameter of 32mm, a bob diameter = 29.5mm and a bob length of 44.25mm, is used when conducting viscosity measurements. Viscosity was measured at room temperature (approximately 23°C). The procedures for measuring viscosity are as follows:

1. Pour polymer solution into the cup;
2. Lower the testing stage attached to the bob to the down position by releasing the auto stage switch;
3. Lower the bob using manual stage control into the cup;
4. Lower the bob into the cup until the polymer solution level reaches the upper surface of the bob; When properly loaded, the polymer solution should appear as shown in the figure.
5. Adjust the shear rate of the rotation to be equal to 100/sec, take the viscosity reading after 1.5 min or when viscosity reading becomes stable.

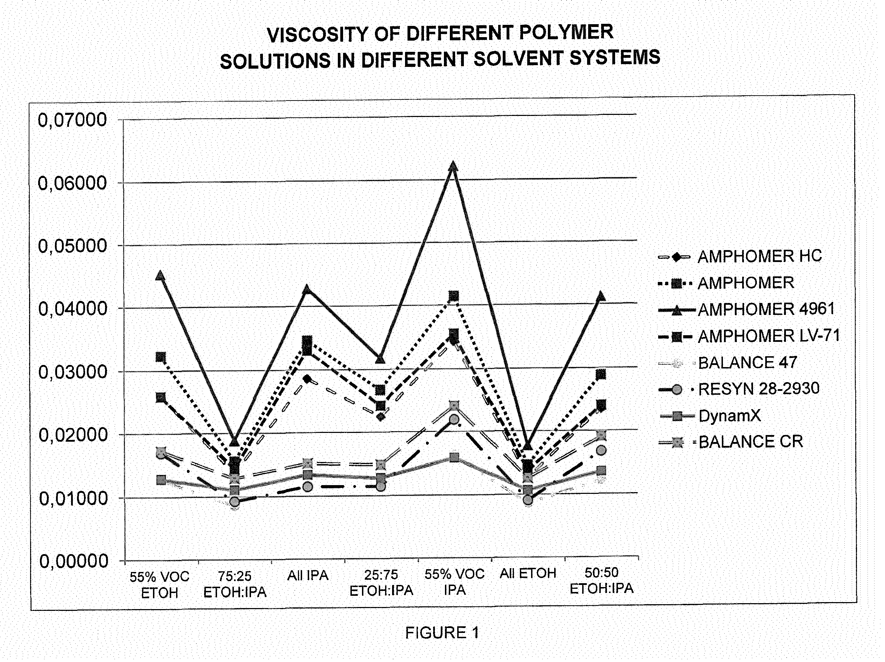

The results of the viscosity measurements are included in Table 3 and are shown in Figure 1.

Table 3

| SOLVENT SYSTEM | AMPHOMER HC | AMPHOMER | AMPHOMER 4961 | AMPHOMER LV-71 | BALANCE 47 | RESYN 28-2930 | DynamX | BALANCE CR |
|---|---|---|---|---|---|---|---|---|
| **55% VOC ETOH** | 0.02591 | 0.03235 | 0.04532 | 0.02588 | 0.01271 | 0.01691 | 0.012845 | 0.01723 |
| **75:25 ETOH:IPA** | 0.01362 | 0.01556 | 0.01880 | 0.01438 | 0.00856 | 0.00927 | 0.011088 | 0.01287 |
| **All IPA** | 0.02854 | 0.03465 | 0.04279 | 0.03308 | | 0.01152 | 0.013390 | 0.01518 |
| **25:75 ETOH:IPA** | 0.02250 | 0.02670 | 0.03178 | 0.02424 | | 0.01150 | 0.012799 | 0.01488 |
| **55% VOC IPA** | 0.03440 | 0.04145 | 0.06221 | 0.03551 | 0.01635 | 0.02195 | 0.015879 | 0.02411 |
| **All ETOH** | 0.01269 | 0.01464 | 0.01771 | 0.01371 | 0.00865 | 0.00908 | 0.010677 | 0.01259 |
| **50:50 ETOH:IPA** | 0.02349 | 0.02882 | 0.04119 | 0.02404 | 0.01225 | 0.01676 | 0.013605 | 0.01907 |

As shown, the viscosities of the compositions comprising ethanol and isopropanol were at or below about 0.060 pascal seconds.

Example 3-Determination of Turbidity

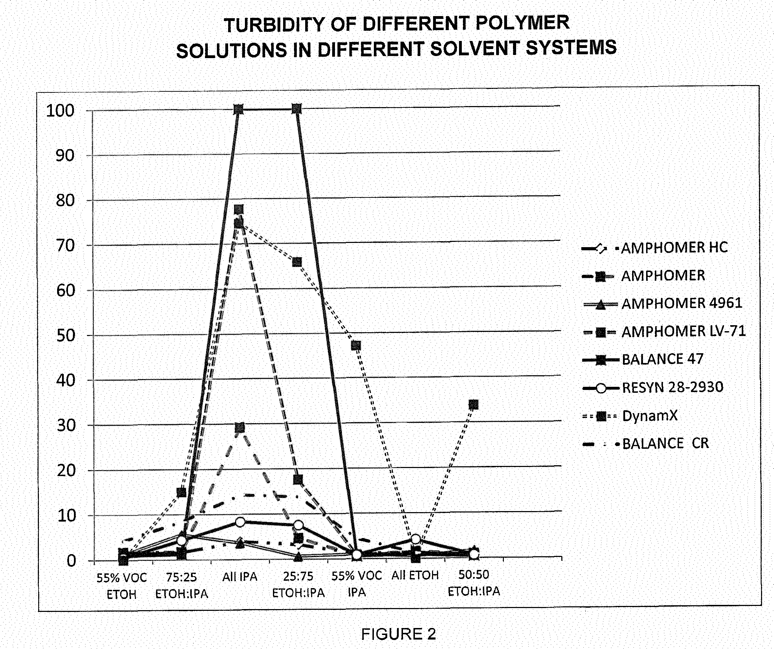

Samples of formulations containing several different hair fixative polymers in various solvent systems were tested in order to determine the turbidity of the sample formulations. The samples were placed in a HACH tube (glass tubes designed for the specific instrument). The samples were then measured on a HACH Turbidimeter (model number 2100N) and reported in NTU. Turbidity was measured at room temperature (approximately 23°C). The results of the turbidity measurements are shown in Table 4 and graphically in Figure 2.

Table 4

| SOLVENT SYSTEM | AMPHOMER HC | AMPHOMER | AMPHOMER 4961 | AMPHOMER LV-71 | BALANCE 47 | RESYN 28-2930 | DynamX | BALANCE CF |
|---|---|---|---|---|---|---|---|---|
| 55°/ VOC ETOH | 1.69 | 1.68 | 1.13 | 1.38 | 0.915 | 0.465 | 0 | 4.21 |
| 75:25 ETOH:IPA | 1.67 | 1.71 | 5.55 | 1.55 | 1.22 | 4.28 | 15 | 8.4 |
| All IPA | 3.95 | 29.2 | 3.69 | 77.7 | 100 | 8.34 | 74.54 | 14.3 |
| 25:75 ETOH:IPA | 3.25 | 4.62 | 0.66 | 17.6 | 100 | 7.52 | 65.9 | 13.8 |
| 55°/ VOC IPA | 1.13 | 0.837 | 1.09 | 0.77 | 0.547 | 0.855 | 47.14 | 4.45 |
| All ETOH | 1.35 | 1.54 | 0.57 | 1.31 | 0.97 | 4.2 | 0 | 1.23 |
| 50:50 ETOH:IPA | 0.69 | 0.99 | 1.73 | 0.86 | 0.503 | 0.733 | 33.75 | 0.944 |

As shown in Table 4 and Figure 2, the turbidity of the compositions comprising ethanol and isopropanol were at or below about 30 NTU or less.

Example 4 - Determination of Surface Tension

Samples of formulations containing several different hair fixative polymers in various solvent systems were tested in order to determine the surface tension of the sample formulations. The samples tested included solvent systems of formula numbers 8-14, as identified in Table 5:

Table 5

| SOLVENT SYSTEM | Formula Number |
|---|---|
| 55% VOC ETOH | 8 |
| 75:25 ETOH:IPA | 9 |
| All IPA | 10 |
| 25:75 ETOH:IPA | 11 |
| 55% VOC IPA | 12 |
| All ETOH | 13 |
| 50:50 ETOH:IPA | 14 |

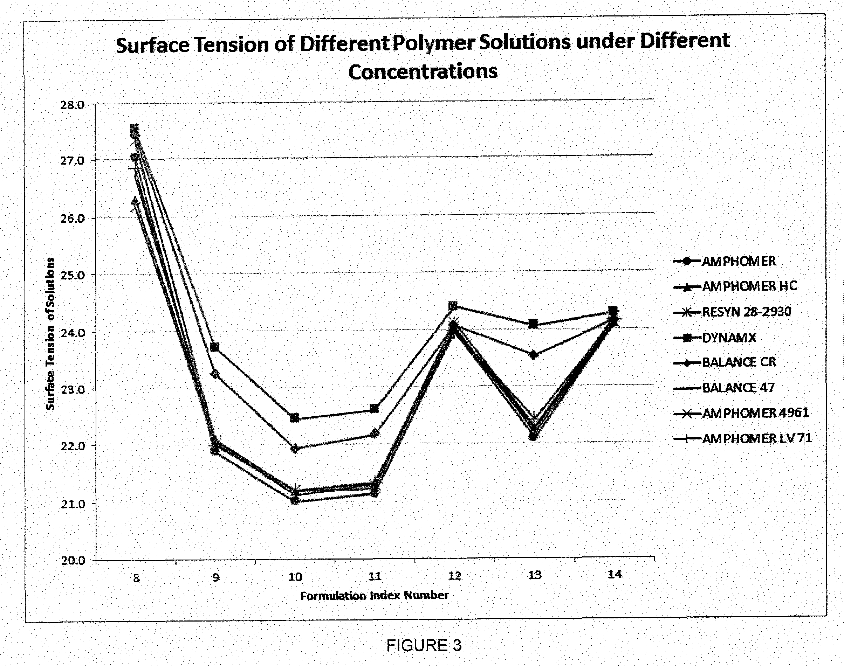

Surface Tension was measured using a Kruss SF2000 micro balance with a wimbley plate attachment. Surface Tension was measured at room temperature (approximately 23°C). The results of the surface tension measurements are shown in Figure 3. The Surface Tension graph shows the data for all the polymers tested at 7% polymer solids in the same solvent systems examined above for performance. The surface tension data shows that samples containing IPA have lower surface tension than those including only ethanol. For example, as shown, the surface tension of the compositions comprising ethanol and isopropanol were at or below about 28 Dynes/cm.

Example 5 - Determination of Capillary Number

[0037]    Samples of formulations containing several different hair fixative polymers in various solvent systems were tested in order to determine the capillary number of the sample formulations. The solvent systems were tested at room temperature (approximately 23°C). Capillary number correlates the relationship between viscosity and surface tension using the following formula:

$$\frac{1}{d[4,3]} = 0.018917 - 8.55333\left(\frac{\text{Viscosity}}{\text{Surface Tension}}\right)$$

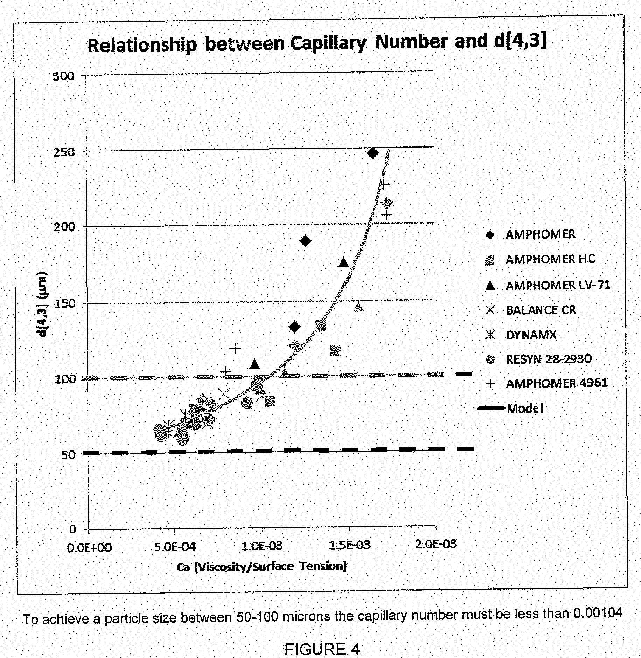

where $d[4,3]$ is the mean particle size of the atomized fixative composition and solvent. The results of the capillary number measurements are shown in Figure 4. Generally, the particle size for hair spray fixative compositions is from about 50 to about 100 microns. The data shows that a capillary number of about 0.00104 or less corresponds to an average particles size of from about 50 to about 100 microns.

Example 6 - Determination of Mean Particle Size

Samples of formulations containing several different hair fixative polymers in various solvent systems were tested in order to determine the mean particle size of the sample formulations. Particle Size was measured on the formulations as delivered from the aerosol can or pump spray. Particle Size was measured on a Malvern Particle Size Analyzer Spraytec® 2600 droplet and particle size analyzer available from Malvern Instruments LMT, Worcestershire, UK. The particle size analyzer was run as follows:

Using the Malvern particle size analyzer (Spraytec® 2600), the samples were prepared as in Table 1 (aerosol formulations). The operation of this instrument is detailed in the attendant instruction manual. The polymer was present at 5% in all Examples and was neutralized with 0.96% AMP. The formulation was charged into an aluminum aerosol can and fitted with the following valve and actuator:

Valve Type: VX-81
Stem Orifice: 0.011 ORFICE VIRGIN NYLON
Body Orifice: 0.010 NO VT
Gasket: 0.045 BUTYL CODE 501
Spring: STAINLESS STEAL 0.018 OPEN C
Cup: T-SEAL HI-PRO LAM EPON TOP DIM
Tube: 0.122 ID
Tube Length: 09 00/16"
Actuator: 0.023" MISTY

which is available from AptarGroup Inc, of Cary, Illinois. The can was placed 8-10 inches from the laser beam and the actuator bottom on the aerosol can was pressed. Samples were run in triplicate and the average for each of the results is reported in Table 6 for each of the solvent systems and polymers. This test was run at room temperature (approx. 23°C) with no control of humidity. The instrument automatically performed the measurements and calculations in accordance with its standard operation.

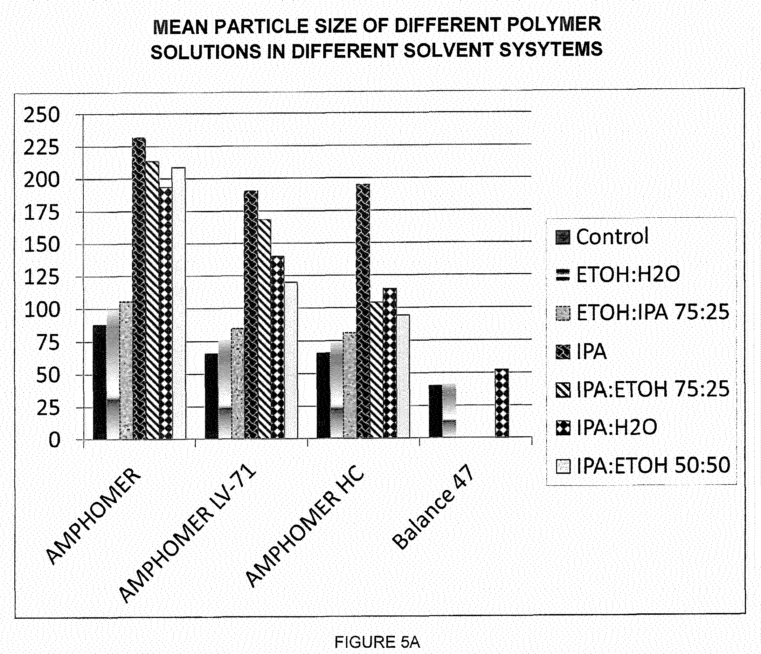

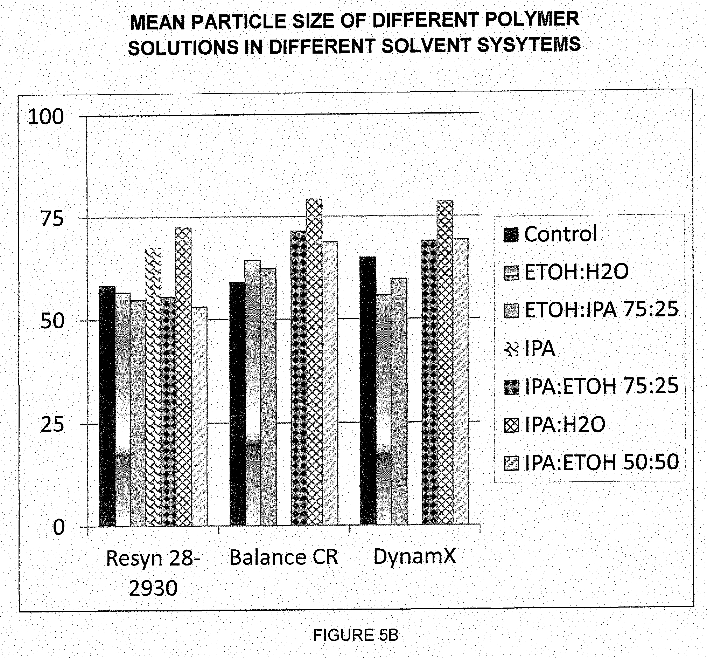

Particle size is a key performance property to consider when developing a hairspray formulation. Particle size is a combination of the solubility of the polymer in the solvents, the viscosity of the solvent, and the surface tension of the solvent. The capillary number marries all three of these properties together to predict particle size. If the spray is too small it dries before it hits the hair creating no hold. If the particles are too large the sprays take too long to dry and weigh the hair down. The results of the mean particle size measurements are shown in Table 6, which are graphically represented in Figure 5.

Table 6

|  | Control | ETOH:H2O | ETOH:IPA 75:25 | IPA | IPA:ETOH 75:25 | IPA:H2O | IPA:ETOH 50:50 |
|---|---|---|---|---|---|---|---|
| AMPHOMER | 87.66 | 99.69 | 105.4 | 232 | 213.24 | 193.23 | 208.5 |
| AMPHOMER LV-71 | 65.59 | 76.33 | 84.79 | 190 | 168.3 | 139.7 | 119.8 |
| AMPHOMER HC | 65.54 | 73.58 | 80.98 | 195 | 104.0 | 114.5 | 94.09 |
| Balance 47 | 39.95 | 41.44 |  |  |  | 52.32 |  |
| Resyn 28-2930 | 58.2 | 56.65 | 54.84 | 67.6 | 55.63 | 72.51 | 53.06 |
| Balance CR | 59.06 | 64.38 | 62.4 |  | 71.46 | 79.28 | 68.74 |
| DynamX | 64.91 | 55.84 | 59.72 |  | 68.91 | 78.64 | 69.22 |

As shown, the mean particle size of the compositions comprising ethanol and isopropanol were from about 50 to about 100 microns.

Example 7 - Subjective Performance Data

In subjective on hair properties the three properties that measure overall hold are stiffness, spring, and webbing. Stiffness is measured by a panelist choosing the swatch that is harder feeling or stiffer. Spring and webbing are a measure of the cohesive and adhesive properties of the polymer with the hair and the polymer. In subjective testing the panelists are asked to choose the swatch with more webbing and the swatch that springs back the fastest and closest to its original shape.

Paired comparisons were done for all the polymers in various solvent systems. All the various solvent systems were compared to the control. Significance was reported at the 95% confidence level. The data is reported in the number of times the test sample was chosen. If the sample is chosen 0 or 1 time the sample is said to perform inferior to the control. If the sample is chosen 7 or 8 times it is said to perform superior to the control. The polymers and solvent systems were compared based on:

**Subjective Properties Description:**

**Beading:**

Visually examine the swatch for dried polymer beads. Choose the swatch which has more beading.

**Gloss:**

Gently handle the swatches so as not to break the films. Visually inspect the swatches to determine which has **more** shine/gloss.

**Stiffness:**

Gently handle swatches and feel for differences in stiffness. Using two fingers, hold the middle of the swatch in a horizontal position - does one bend more than the other? Choose the one that is **more** rigid.

**Spring:**

While holding the swatch in one hand, gently pull on an edge with the other hand three times only. Look for spring back, and bounce. The more elastic the better the Spring.

**Webbing:**

While holding the swatch in both hands, gently pull outward on the edges approx. 4". (Do this three times only to avoid damage to the bonds. If the bonds are destroyed then the dry combing may appear to be easier to comb). The **more** net like the better the Webbing.

**Dry Comb:**

Comb through each swatch (5) times and evaluate ease of combing. Choose the one that combs **more** easily.

**Flake:**

Visually inspect both swatches after combing. Check the teeth of the comb for flake accumulation. Holding the swatch at the bound end run your fingernail down the length of the tress then inspect. Choose the one with **more** flakes.

**Anti-Stat:**

Holding swatch at bound end comb through vigorously 10 times then evaluate for extent of fly aways generated. Choose the one with **more** fly aways.

**Feel:**

Handle swatches and determine preference. Choose the one that feels **more** silky / cleaner.

| **0/8 - 1/8:** | Statistically inferior | **2/8 -6/8:** | Statistically not different |
|---|---|---|---|
| **7/8 - 8/8**: | Statistically superior | | |

The results of the subjective performance tests are demonstrated in Tables 7 and 8. Table 7 demonstrates the results of paired comparisons of AMPHOMER® polymer in different solvent blends compared to an all ethanol solvent system.

Table 7

| | ETOH:H2O | ETOH:IPA 75:25 | IPA | IPA:ETOH 75:25 | IPA:H2O | IPA:ETOH 50:50 |
|---|---|---|---|---|---|---|
| **Beading** | 7 | 3 | 6 | 4 | 3 | 3 |
| **Gloss** | 3 | 5 | 6 | 5 | 3 | 5 |

(continued)

|  | ETOH:H2O | ETOH:IPA 75:25 | IPA | IPA:ETOH 75:25 | IPA:H2O | IPA:ETOH 50:50 |
|---|---|---|---|---|---|---|
| **Stiffness** | 7 | 8 | 3 | 7 | 7 | 7 |
| **Spring** | 4 | 7 | 1 | 4 | 5 | 4 |
| **Webbing** | 4 | 6 | 4 | 3 | 4 | 3 |
| **Dry Comb** | 0 | 1 | 7 | 2 | 1 | 1 |
| **Anti-Stat** | 4 | 4 | 4 | 5 | 2 | 4 |
| **Flake** | 6 | 8 | 2 | 6 | 6 | 7 |
| **Feel** | 1 | 1 | 6 | 2 | 0 | 3 |

As shown in Table 7, the results were as follows:

IPA:ETOH 50:50: Tested superior for stiffness, and inferior for dry comb, and flake
IPA:H20: Tested superior for stiffness and inferior for dry comb
IPA:ETOH 75:25: Tested superior for stiffness
IPA: Tested superior in dry comb and inferior for spring
ETOH:IPA 75:25: Tested superior for stiffness, spring, and inferior for feel, flake
ETOH:H2O: Tested superior for stiffness, spring, and inferior for feel, flake

Table 8 demonstrates the results of paired comparisons of AMPHOMER® LV-71 polymer in different solvent blends compared to an all ethanol solvent system.

Table 8

|  | ETOH:H2O | ETOH:IPA 75:25 | IPA | IPA:ETOH 75:25 | IPA:H2O | IPA:ETOH 50:50 |
|---|---|---|---|---|---|---|
| **Beading** | 6 | 5 | 8 | 5 | 4 | 3 |
| **Gloss** | 5 | 5 | 7 | 3 | 7 | 7 |
| **Stiffness** | 5 | 7 | 7 | 7 | 6 | 8 |
| **Spring** | 4 | 7 | 8 | 7 | 7 | 7 |
| **Webbing** | 5 | 6 | 4 | 7 | 6 | 5 |
| **Dry Comb** | 2 | 4 | 1 | 1 | 1 | 3 |
| **Anti-Stat** | 4 | 3 | 5 | 4 | 3 | 6 |
| **Flake** | 3 | 5 | 6 | 6 | 8 | 7 |
| **Feel** | 5 | 3 | 0 | 2 | 0 | 1 |

As shown in Table 8, the results were as follows:

IPA:ETOH 50:50: Tested superior for stiffness, spring, and inferior for feel, and flake
IPA:H20: Tested superior for spring, gloss and inferior for flake, feel, and dry comb
IPA:ETOH 75:25: Tested superior for stiffness, spring, webbing
IPA: Tested superior in stiffness, spring, gloss and inferior for feel, dry comb, beading
ETOH:IPA 75:25: Tested superior for stiffness, spring
ETOH:H2O: Tested equivalent for all performance properties

Example 8 - Determination of High Humidity Curl Retention (HHCR)

The high humidity curl retention properties of hair styling compositions of the present invention were measured for compositions formulated with RESYN® polymers and AMPHOMER® polymers in various solvent systems. The test was conducted at 72°F (22°C) and 90% Relative Humidity over a period of 24 hours. The test was performed on 10" long x 2-gram swatches of European virgin brown hair (9 replicate swatches per sample). Curl retention testing is run in a humidity chamber set at 70°F/90% Relative Humidity for a total of 24 hours. Readings for %, Curl Retention are read and recorded at time intervals of 15, 30, 60, 90 min, 2, 3, 4, 5, and 24 hrs. The hair styling compositions were tested according to the following procedures:

1. Wet hair swatch, comb through to remove tangles and squeeze out excess water (run swatch between thumb and index finger).

2. Apply sample to swatch, gently "work into" swatch and comb through.

3. Roll swatch on 1/2" diameter Teflon mandrel. Carefully remove rolled swatch from mandrel and secure with two hair clips.

4. Place curls on tray and dry in oven overnight.

5. Remove dried curls from oven and let cool to room temperature.

6. Suspend curls, from bound end of swatch, on graduated clear, transparent curl retention boards.

7. Remove clips from curls and gently unwind with glass rod making sure to "break" the curl.

8. Take initial curl length readings before placing boards and curls into environmental chamber (70°F, 90% relative humidity).

9. Record curl lengths at the 15, 30, 60, 90, 2, 3, 4, 5, and hour time intervals.

10. At conclusion of test, remove boards and curls from chamber.

11. Clean used hair swatches.

12. Calculate % Curl Retention and comparison of samples.

The Samples were prepared as follows:

HHCR was run in a constant temperature and humidity chamber. Curls were rolled on a mandrel and allowed to dry overnight. The curls were then sprayed with the polymer solutions and allowed to dry. Then the curls were hung on a board placed in the oven and the percent of curl loss was tracked over 24 hrs.

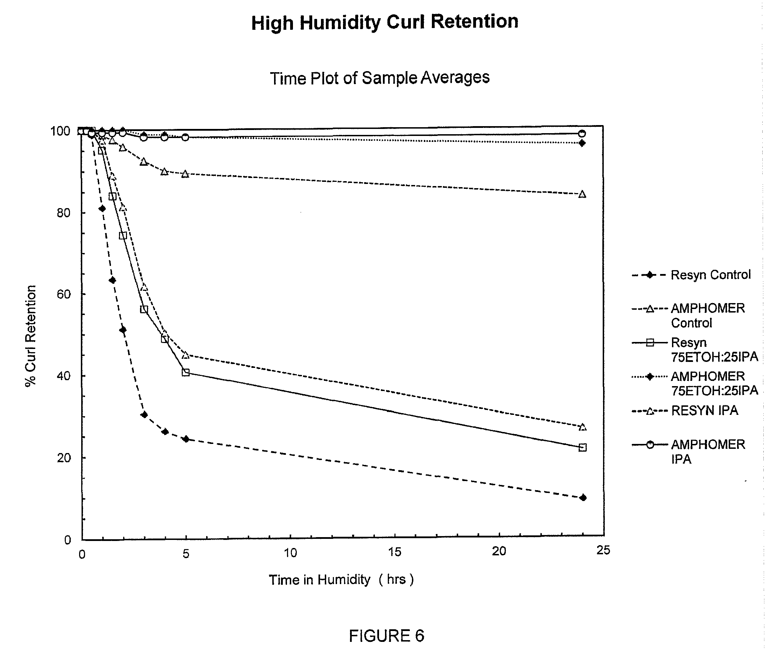

The results in Table 9, which are shown graphically in Figure 6, represent the HHCR data for the RESYN® 28-2930 and AMPHOMER® polymers in various solvent systems.

TABLE 9

| Formula | 5hrs | 24hrs |
|---|---|---|
| RESYN® 28-2930 polymer Control (in ETOH) | 24.37% | 9.22% |
| AMPHOMER® polymer Control (in ETOH) | 89.44% | 83.59% |
| RESYN® 28-2930 polymer in IPA | 44.96% | 26.54% |
| AMPHOMER® polymer in IPA | 98.21% | 98.21 % |
| RESYN® 28-2930 polymer in 75:25 ETOH:IPA | 40.62% | 21.45% |
| AMPHOMER® polymer in 75:25 ETOH:IPA | 98.21% | 95.87% |

Surprisingly, the data shows that compared to ethanol-only solvent systems, solvent systems having a ratio of 75:25 ethanol to isopropanol had much higher high humidity curl retention than would have been expected. As seen in Table 9, the samples that included 75:25 ethanol to isopropanol solvent systems exhibited high humidity curl retention that were much closer to and representative of isopropanol-only solvent systems. Further, after 24 hrs at 21.1 °C and 90% relative humidity, the AMPHOMER® polymer samples with isopropanol showed superior performance to the AMPHOMER® polymer control with ethanol only and the RESYN® 28-2930 polymer samples with isopropanol showed superior performance to the RESYN® 28-2930 polymer control sample with just ethanol at the 95% confidence level.

As can be seen from the data, the inclusion of isopropanol in combination with ethanol, particularly in ratios above 50:50 ethanol to isopropanol, exhibited unexpected improvements in stiffness, webbing and spring over ethanol-only systems. Further, the solvent systems of the invention further resulted in high humidity curl retention better than expected high humidity curl retention data particularly in ratios of 75:25 ethanol to isopropanol. That is, the inclusion of isopropanol was expected to result in the loss of hydrophobicity of the film and thus result in poorer high humidity curl retention.

Example 9 - Determination of Polymer Film Elongation

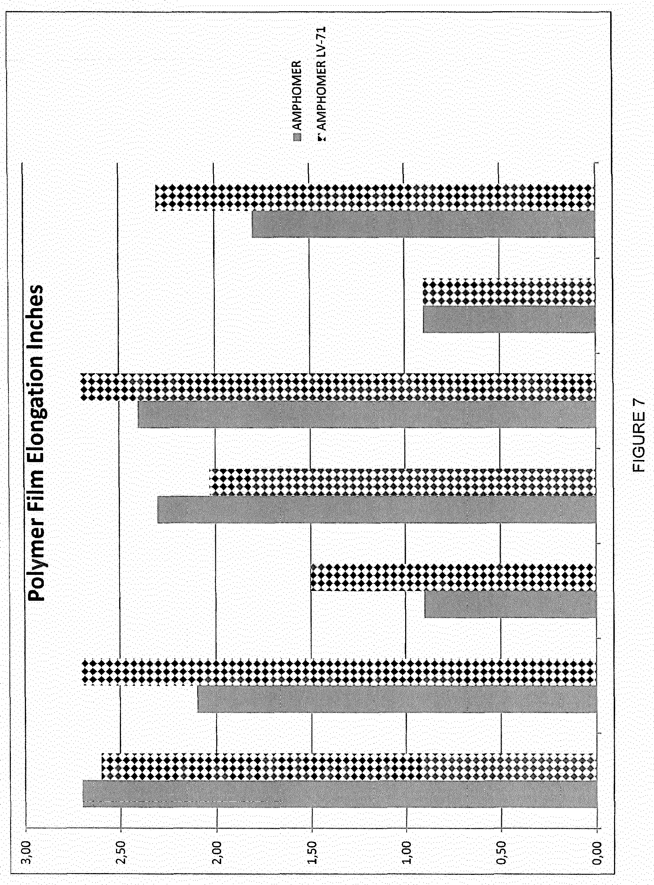

Samples of different polymer films resulting from polymer compositions of two different AMPHOMER® hair fixative polymers (AMPHOMER® and AMPHOMER® LV-71) initially prepared in solvent systems of ethanol and/or isopropanol of varying concentrations were tested in order to determine the polymer elongation of the film, in inches. 15% solids polymer solutions are prepared and placed in a 1mm silicone mold. They were allowed to dry for 24 hrs at 23°C 50% RH. After 24 hrs the films were removed from the molds, measured for thickness on an air micrometer and then pulled using a Sintech micro balance. The results of the polymer elongation measurements are shown in Figure 7. The data shows that the polymer film for both AMPHOMER® and AMPHOMER® LV-71 polymers provided substantial increases in elongation when isopropanol is present in the formulation when compared to the AMPHOMER® polymer in the ethanol solvent control. This was seen across the range of ratios of ethanol to isopropanol in the samples tested, as seen in Figure 7. This was considered surprising, considering that the films were dry (i.e. the solvent had evaporated and was no longer present) and it was not expected that the selection of solvent systems would have any significant impact on the dry film.

While particular embodiments of the present invention have been illustrated and described herein, the invention is not intended to be limited to the details shown.

**Claims**

1.  A hair fixative composition comprising:

    at least one fixative polymer; and
    a solvent system comprising ethanol and at least one other non-aqueous solvent chosen from isopropanol, n-propanol or a combination thereof in a weight ratio in a range of about 80:20 to about 20:80 of ethanol to the at least one other non-aqueous solvent
    wherein the at least one fixative polymer is selected from the group consisting of octylacrylamide/acrylates/butylaminoethyl methacrylate copolymer), acrylates/octylacrylamide copolymer, acrylates copolymer, octylacrylamide/butylaminoethyl methacrylate copolymer, VA/crotonates/vinyl neodecanoate copolymer and polyurethane-14 (and) AMP-Acrylates copolymer.

2.  The hair fixative composition of claim 1 wherein the weight ratio of ethanol to the at least one other non-aqueous solvent is in a range of about 75:25 to about 25:75.

3.  The hair fixative composition of claims 1 or 2 wherein solvent system is present in the hair fixative composition in about 40% to about 98% by weight of the hair fixative composition.

4.  The hair fixative composition of claims 1 to 3 wherein the composition further comprises water.

5.  The hair fixative composition according to any of the preceding claims wherein the composition has a maximum incremental reactivity of about 0.80 or less.

6.  The hair fixative composition according to any of the preceding claims wherein the at least one fixative polymer comprises at least one monomer selected from the group consisting of acrylic acid, methacrylic acid, methylacrylate, methacrylate, vinyl acetate, crotonic acid, vinyl neodecanate, isocyanate and t-octylacrylamide.

7.  The hair fixative composition according to any of the preceding claims wherein the at least one fixative polymer is present in an amount of 0.1 to 10% by weight of the composition.

8.  The hair fixative composition according to any of the preceding claims wherein the composition has a viscosity of about 0.10 Pascal second or less.

9.  The hair fixative composition of any of the preceding claims wherein the composition has a turbidity of about 30 NTU or less.

10.  The hair fixative composition according to any of the preceding claims further comprising a neutralizing agent.

11.  The hair fixative composition according to any of the preceding claims further comprising a propellant.

12.  The hair fixative composition of claim 11 wherein the propellant is selected from the group consisting of dimethyl ether, one or more $C_3$-$C_6$ straight and branched chain hydrocarbons, halogenated hydrocarbons and a compressed gas.

**Patentansprüche**

1. Haarfestiger-Zusammensetzung umfassend:

   mindestens ein festigendes Polymer; und
   ein Lösungsmittelsystem umfassend Ethanol und mindestens ein weiteres, nicht wässriges Lösungsmittel, ausgewählt aus Isopropanol, n-Propanol oder einer Kombination daraus, in einem Gewichtsverhältnis in einem Bereich von etwa 80:20 bis etwa 20:80 von Ethanol zu dem mindestens einen weiteren, nicht wässrigen Lösungsmittel,
   wobei das mindestens eine festigende Polymer aus der Gruppe ausgewählt ist, bestehend aus Octylacrylamid/Acrylaten/Butylaminoethylmethacrylat-Copolymer, Acrylaten/Octylacrylamid-Copolymer, Acrylate-Copolymer, Octylacrylamid/Butylaminoethylmethacrylat-Copolymer, VA/Crotonate/Vinylneodecanoat-Copolymer und Polyurethan-14 (und) AMP-Acrylate-Copolymer.

2. Haarfestiger-Zusammensetzung nach Anspruch 1, wobei das Gewichtsverhältnis von Ethanol zu dem mindestens einen weiteren, nicht wässrigen Lösungsmittel in einem Bereich von etwa 75:25 bis etwa 25:75 liegt.

3. Haarfestiger-Zusammensetzung nach Anspruch 1 oder 2, wobei das Lösungsmittelsystem in der Haarfestiger-Zusammensetzung in etwa 40 bis etwa 98 Gew.-% der Haarfestiger-Zusammensetzung vorhanden ist.

4. Haarfestiger-Zusammensetzung nach Anspruch 1 bis 3, wobei die Zusammensetzung weiterhin Wasser umfasst.

5. Haarfestiger-Zusammensetzung nach einem der vorhergehenden Ansprüche, wobei die Zusammensetzung eine maximale inkrementelle Reaktivität von etwa 0,80 oder weniger hat.

6. Haarfestiger-Zusammensetzung nach einem der vorhergehenden Ansprüche, wobei das mindestens eine festigende Polymer mindestens ein Monomer, ausgewählt aus der Gruppe, bestehend aus Acrylsäure, Methacrylsäure, Methylacrylat, Methacrylat, Vinlyacetat, Crotonsäure, Vinylneodecanat, Isocyanat und t-Octylacrylamid, umfasst.

7. Haarfestiger-Zusammensetzung nach einem der vorhergehenden Ansprüche, wobei das mindestens eine festigende Polymer in einer Menge von 0,1 bis 10 Gew.-% der Zusammensetzung vorhanden ist.

8. Haarfestiger-Zusammensetzung nach einem der vorhergehenden Ansprüche, wobei die Zusammensetzung eine Viskosität von etwa 0,10 Pascalsekunden oder weniger hat.

9. Haarfestiger-Zusammensetzung nach einem der vorhergehenden Ansprüche, wobei die Zusammensetzung eine Trübung von etwa 30 NTU oder weniger hat.

10. Haarfestiger-Zusammensetzung nach einem der vorhergehenden Ansprüche, weiterhin umfassend ein Neutralisationsmittel.

11. Haarfestiger-Zusammensetzung nach einem der vorhergehenden Ansprüche, weiterhin umfassend ein Treibmittel.

12. Haarfestiger-Zusammensetzung nach Anspruch 11, wobei das Treibmittel aus der Gruppe ausgewählt ist, bestehend aus Dimethylether, einem oder mehreren $C_3$-$C_6$ geradkettigen und verzweigtkettigen Kohlenwasserstoffen, halogenierten Kohlenwasserstoffen und einem komprimierten Gas.

**Revendications**

1. Composition de fixateur pour cheveux comprenant :

   au moins un polymère fixateur ; et
   un système de solvant comprenant de l'éthanol et au moins un autre solvant non aqueux choisi parmi l'isopropanol, le n-propanol ou une combinaison de ceux-ci selon un rapport pondéral dans une plage allant d'environ 80:20 à environ 20:80 de l'éthanol sur l'au moins un autre solvant non aqueux
   dans laquelle l'au moins un polymère fixateur est choisi dans le groupe constitué de copolymère d'octylacrylamide/acrylates/méthacrylate de butylaminoéthyle, de copolymère d'acrylates/octylacrylamide, de copolymère d'acrylates, de copolymère d'octylacrylamide/méthacrylate de butylaminoéthyle, de copolymère de VA/crotonates/néodécanoate de vinyle et de copolymère de polyuréthane-14 (et) d'AMP-acrylates.

2. Composition de fixateur pour cheveux de la revendication 1, dans laquelle le rapport pondéral de l'éthanol sur l'au moins un autre solvant non aqueux se trouve dans une plage allant d'environ 75:25 à environ 25:75.

3. Composition de fixateur pour cheveux des revendications 1 ou 2, dans laquelle le système de solvant est présent dans la composition de fixateur pour cheveux en une quantité allant d'environ 40% à environ 98% en poids de la composition de fixateur pour cheveux.

4. Composition de fixateur pour cheveux des revendications 1 à 3, dans laquelle la composition comprend en outre de l'eau.

5. Composition de fixateur pour cheveux selon l'une des revendications précédentes, dans laquelle la composition présente une réactivité incrémentale maximale inférieure ou égale à environ 0,80.

6. Composition de fixateur pour cheveux selon l'une des revendications précédentes, dans laquelle l'au moins un polymère fixateur comprend au moins un monomère choisi dans le groupe constitué d'acide acrylique, d'acide méthacrylique, de méthylacrylate, de méthacrylate, d'acétate de vinyle, d'acide crotonique, de néodécanoate de vinyle, d'isocyanate et de t-octylacrylamide.

7. Composition de fixateur pour cheveux selon l'une des revendications précédentes, dans laquelle l'au moins un polymère fixateur est présent en une quantité allant de 0,1 à 10% en poids de la composition.

8. Composition de fixateur pour cheveux selon l'une des revendications précédentes, dans laquelle la composition présente une viscosité inférieure ou égale à environ 0,10 Pascal-seconde.

9. Composition de fixateur pour cheveux de l'une des revendications précédentes, dans laquelle la composition présente une turbidité inférieure ou égale à environ 30 NTU.

10. Composition de fixateur pour cheveux selon l'une des revendications précédentes, comprenant en outre un agent neutralisant.

11. Composition de fixateur pour cheveux selon l'une des revendications précédentes, comprenant en outre un agent propulseur.

12. Composition de fixateur pour cheveux de la revendication 11, dans laquelle l'agent propulseur est choisi dans le groupe constitué de l'éther diméthylique, d'un ou de plusieurs hydrocarbure(s) en $C_3$ à $C_6$ à chaîne droite ou ramifiée, d'hydrocarbures halogènes et d'un gaz comprimé.

EP 2 753 298 B1

## VISCOSITY OF DIFFERENT POLYMER SOLUTIONS IN DIFFERENT SOLVENT SYSTEMS

FIGURE 1

EP 2 753 298 B1

FIGURE 2

FIGURE 3

EP 2 753 298 B1

FIGURE 4

To achieve a particle size between 50-100 microns the capillary number must be less than 0.00104

MEAN PARTICLE SIZE OF DIFFERENT POLYMER
SOLUTIONS IN DIFFERENT SOLVENT SYSYTEMS

Legend:
- Control
- ETOH:H2O
- ETOH:IPA 75:25
- IPA
- IPA:ETOH 75:25
- IPA:H2O
- IPA:ETOH 50:50

Categories: AMPHOMER, AMPHOMER LV-71, AMPHOMER HC, Balance 47

FIGURE 5A

EP 2 753 298 B1

MEAN PARTICLE SIZE OF DIFFERENT POLYMER
SOLUTIONS IN DIFFERENT SOLVENT SYSYTEMS

Legend:
- Control
- ETOH:H2O
- ETOH:IPA 75:25
- IPA
- IPA:ETOH 75:25
- IPA:H2O
- IPA:ETOH 50:50

X-axis: Resyn 28-2930, Balance CR, DynamX

FIGURE 5B

EP 2 753 298 B1

# High Humidity Curl Retention

## Time Plot of Sample Averages

FIGURE 6

EP 2 753 298 B1

FIGURE 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 201011695 A

- EP 2213333 A